# EUROPEAN PATENT APPLICATION

(11) **EP 1 803 812 A1**
(43) Date of publication of application: **04.07.2007**
(21) Application number: 05799379.2
(22) Date of filing: 28.10.2005
(51) Int. Cl.: C12N 15/09, C12N 1/19, C12Q 1/02

(54) **METHOD OF GENE SCREENING WITH YEAST HAVING ERGOSTEROL SYNTHASE UNDERGOING INDUCIBLE EXPRESSION**

(30) Priority: 29.10.2004 JP 2004315611
(71) Applicant: Eisai R&D Management Co., Ltd., Tokyo 112-8088 (JP)
(72) Inventor: MITSUHASHI, Kaoru, c/o EISAI CO., LTD., Tsukuba-shi, Ibaraki 3002635 (JP); TSUKAHARA, Kappei c/o EISAI CO., LTD., Tsukuba-shi, Ibaraki 3002635 (JP)
(74) Representative: Dean, John Paul
(86) International application number: PCT/JP2005/019895
(87) International publication number: WO 2006/046694

(57) **Abstract**

An expression of an ergosterol biosynthetic enzyme such as ERG6 and ERG3 is regulated with an inducible promoter, and transformation is performed in a state where the enzyme is expressed, and then screening or assay of drug-resistant colonies is performed in a state where the expression of the enzyme is repressed.

## Description

### Technical Field

The present invention relates to a method of screening a drug target gene using yeast that inducibly expresses ergosterol biosynthetic enzyme.

### Background Art

The multicopy suppressor method is known as a method of screening a drug target gene using yeast. In the multicopy suppressor method, when expression of a phenotype caused by a drug is suppressed by a gene introduced in multiple copies, the gene is selected as a candidate for a target gene of the drug. When yeast exhibits a phenotype such as lethality or growth inhibition against a drug, yeast into which a gene has been introduced is cultured in the presence of the drug, and the target gene of the drug is identified from yeast that can grow.
However, a drug is often ineffective due to the function of a drug efflux pump and the drug permeability is low due to specific cell walls of yeast, which makes it difficult to search a drug target gene using yeast.
With regard to a drug efflux pump, it has been reported that drug sensitivity can be increased by deleting transcription factors Pdr1p and Pdr3p, which induce expression of a drug efflux pump such as Pdr5p (Non-Patent Document 1). In addition to the deletion of PDR1 and PDR3, deletion of ERG6, an enzyme in the ergosterol biosynthetic pathway, which is a component of yeast cell walls is known to increase drug permeability and to further increase drug sensitivity. However, an erg6-disrupted strain in which the enzyme is deleted has low transformation efficiency, resulting in difficulty in the introduction of a gene library (Non-Patent Document 2). In particular, in the case of introducing a library comprising many genes such as a mammal gene library into the erg6-disrupted strain, at least millions of transformants would be necessary. However, the procedure is substantially impossible because it is expected that yeast should be cultured in several hundred liters of medium, so that the erg6-disrupted strain is considered to be unsuitable for the multicopy suppressor method.
Non-Patent Document 1: Delaveau T., et. al., Mol Gen Genet., 244(5), 501-511, 1994
Non-Patent Document 2: Gaber RF. et. al., Mol Cell Biol., 9(8), 3447-3456, 1989

### DISCLOSURE OF THE INVENTION

An object of the present invention is to develop yeast in which an enzyme in the ergosterol biosynthetic pathway, in particular, ERG6 is disrupted and which has both properties of high drug permeability and high transformation efficiency and is suitable for screening of a drug target gene.

The inventors of the present invention considered that it is necessary to improve transformation efficiency to use a strain in which a gene of an enzyme in the ergosterol biosynthetic pathway is disrupted and has high sensitivity to a drug for screening of a drug target gene. Deficiency of an enzyme in the ergosterol biosynthetic pathway results in low transformation efficiency, so that the inventors considered that, if expression of the enzyme in the ergosterol biosynthetic pathway is induced only during transformation, high transformation efficiency could be maintained.
The inventors of the present invention have created yeast having higher drug permeability by: regulating expression of an enzyme in the ergosterol biosynthetic pathway using an inducible promoter; maintaining high transformation efficiency by inducing expression of the enzyme in the ergosterol biosynthetic pathway during transformation; and repressing expression of the enzyme in the ergosterol biosynthetic pathway during screening or assay of drug-resistant colonies.
That is, the present invention is as follows.
(1) A method of allowing a compound to interact with a protein in yeast cell, comprising the steps of:
   (i) introducing a gene encoding the protein into yeast to express the protein in a state where expression of an enzyme involved in the ergosterol biosynthetic pathway is induced; and
   (ii) adding the compound to the yeast into which the gene has been introduced to allow the compound to interact with the protein in yeast cell in a state where expression of the enzyme in the ergosterol biosynthetic pathway is repressed.
(2) A method of identifying a gene encoding a protein that interacts with a compound by contacting the compound with the protein expressed in yeast, comprising the steps of:
   (i) introducing a gene library into yeast to express proteins in a state where expression of an enzyme in the ergosterol biosynthetic pathway is induced;
   (ii) adding the compound to the yeast where the gene library has been introduced to contact the compound with the protein in a state where expression of the enzyme in the ergosterol biosynthetic pathway is repressed;
   (iii) selecting yeast whose phenotype has been altered by the interaction between the compound and the protein; and
   (iv) identifying a gene that has been introduced into the selected yeast.
(3) The method according to (2), wherein the phenotype of yeast caused by the compound is lethality or growth inhibition.
(4) The method according to any one of (1) to (3), wherein the induction and the repression of expression of the enzyme in the ergosterol biosynthetic pathway is controlled by transcriptional regulation in the yeast.
(5) The method according to (4), wherein the transcriptional regulation is transcriptional regulation by GAL10 promoter or GAL1 promoter.
(6) The method according to any one of (1) to (5), wherein the enzyme in the ergosterol biosynthetic pathway is selected from ERG6 and ERG3.
(7) Yeast which inducibly expresses ERG6 or ERG3.

### BRIEF DESCRIPTION OF THE DRAWINGS

Fig. 1 shows comparison of cycloheximide-sensitivity of the yeast having ERG6 to be inducibly expressed by galactose in the presence of galactose (B: galactose medium) and in the absence of galactose (A: glucose medium).
Fig. 2 shows comparison of transformation of the yeast having ERG6 to be inducibly expressed by galactose in the presence of galactose (B: galactose medium) and in the absence of galactose (A: glucose medium).

### DESCRIPTION OF THE PREFERRED EMBODIMENTS

An object of the present invention is to provide a method comprising: introducing a gene into yeast in the state where expression of an enzyme in the ergosterol biosynthetic pathway is induced to express a protein; and adding a compound to the yeast in the state where expression of an enzyme in the ergosterol biosynthetic pathway is repressed so that permeability of the compound to yeast cell is high, to thereby allow the compound to interact with the product of the introduced gene.

Yeast that can be used in the present invention is not particularly limited as long as it can be introduced with a gene and can incorporate a compound, and examples thereof include *Saccharomyces cerevisiae* and *Schizosaccharomyces pombe.*

Yeast that can be used in the present invention is preferably one that inducibly expresses an enzyme in the ergosterol biosynthetic pathway.
The enzyme in the ergosterol biosynthetic pathway can be selected from any enzymes as long as the absence of its expression does not lead to lethality. When *Saccharomyces cerevisiae* is used as yeast, the enzyme is preferably Erg3p or Erg6p, more preferably ERG6p. An example of the ERG6p includes one having the amino acid sequence of SEQ ID NO: 5 (GenBank Accession No. CAA89944). Meanwhile, the enzyme may be one having an amino acid sequence of SEQ ID NO: 5 including substitution, deletion, or addition of one or several amino acids as long as it has the activity of the enzyme. In this case, the term "several" refers to, for example, 2 to 20, preferably 2 to 10, more preferably 2 to 5. On the other hand, an example of the Erg3p includes one having the amino acid sequence of SEQ ID NO: 6 (GenBank Accession No. CAA97586). Meanwhile, the enzyme may be one having an amino acid sequence of SEQ ID NO: 6 including substitution, deletion, or addition of one or several amino acids as long as it has the activity of the enzyme. In this case, the term "several" refers to, for example, 2 to 20, preferably 2 to 10, more preferably 2 to 5.
The yeast that can be used in the present invention may be one which inducibly expresses an enzyme in the ergosterol biosynthetic pathway and in which genes encoding Pdr1p and Pdr3p are disrupted.
When *Schizosaccharomyces pombe* is used as yeast, SPAC1687.16c (GenBank: CAA22610) and SPBC16E9.05 (GenBank: CAB 16897) are known as enzymes corresponding to Erg3p and Erg6p, respectively.

The phrase "inducibly express" means that an expression-repressed state and an expression-induced state can be regulated. In the "expression-repressed state", the synthesis of ergosterol may be repressed to such an extent that the drug sensitivity is increased, and for example, preferable is a state in which the expression level of the enzyme is decreased to not more than 10% as compared with that of a wild-type strain or an unmodified strain. On the other hand, in the "expression-induced state", the synthesis of ergosterol may be induced to such an extent that efficiency of gene introduction is increased, and for example, preferable is a state in which the expression level of the enzyme is induced to a level equal to that of a wild-type strain or an unmodified strain, more preferably is a state in which the expression level of the enzyme is induced to 80% or more as compared with that of a wild-type strain or an unmodified strain. The expression level of ergosterol biosynthetic enzyme can be determined by Northern blotting or RT-PCR, for example.

The operations described below can be performed in accordance with an appropriate manual, for example, the methods described in Molecular Cloning: A Laboratory Manual, Cold Spring Harbor, NY, etc.

Examples of a method of regulating expression of an enzyme in the ergosterol biosynthetic pathway include methods of regulating transcription of a gene encoding the enzyme.
Examples of the method of regulating transcription include a method using a tetracycline-regulated gene expression system (Tet System) and a method of ligating a gene encoding an enzyme in the ergosterol biosynthetic pathway to an inducible promoter to express the enzyme.

The Tet System has been developed as a transcription regulatory system in a eukaryote based on properties of a Tet repressor (TetR protein) and a Tet operator sequence (tetO sequence: a DNA sequence upstream of a Tet operon), which act on transcription regulation of an *Escherichia coli* tetracycline-resistant operon. The Tet repressor is a tetR gene product and binds to the tetO sequence only in the absence of tetracycline, and when tetracycline directly binds to the Tet repressor, the Tet repressor cannot bind to the tetO sequence. In the Tet System, the expression level of a target gene integrated downstream of a TRE promoter (a promoter having a Tet responsive element comprising seven repeats of a TetO sequence) can be quantitatively regulated by using a transcriptional activator that is obtained by fusing TetR and a transcriptional activation domain, and by regulating the concentration of tetracycline in a medium, and the system can be applied to yeast (Belli G, et. al., Nucleic Acids Res. 26(4): 942-7. 1998).

When *Saccharomyces cerevisiae* is used as yeast, examples of the inducible promoter include GAL10 promoter (Johnston M and Davis RW. Mol Cell Biol. 4(8): 1440-1448, 1984), GAL1 promoter: (Johnston M and Davis RW. Mol Cell Biol. 4(8): 1440-1448, 1984), CUP1 promoter (Macreadie IG et. al., Plasmid. 21(2): 147-150, 1989), and PHO5 promoter (Meyhack B, et. al., EMBO J. 1(6): 675-680. 1982). Meanwhile, when *Schizosaccharomyces pombe* is used as yeast, an example of the inducible promoter includes nmt1 promoter (Maundrell K., J Biol Chem. 1990 Jul 5; 265(19): 10857-64.).

An example of a specific method to obtain an inducible promoter that functions in yeast is as follows. The nucleotide sequence information of the above-described promoters which are known to be inducible is available from Saccharomyces Genome Database (http://www.yeastgenome.org/). Based on GAL10 promoter (Johnston M and Davis RW. Mol Cell Biol. 4(8): 1440-1448, 1984), GAL1 promoter (Johnston M and Davis RW. Mol Cell Biol. 4(8): 1440-1448, 1984), CUP1 promoter (Macreadie et. al., Plasmid. 21(2): 147-150, 1989), and PHO5 promoter (Meyhack B, et. al., EMBO J. 1(6): 675-680. 1982), primers are designed so that the promoter regions are sandwiched, and amplification is performed by PCR using yeast genomic DNA as a template. Restriction enzyme sites are preliminarily added to the primers, and a restriction enzyme treatment is performed after PCR to ligate the resultant product to a vector based on the restriction enzyme sites. If a marker gene, for example, KanMx6 gene, his5 *(S. pombe)* gene, or the like is preliminarily connected adjacent to the promoters, it can be used as a phenotypic marker when the promoters are introduced into yeast (Butt TR, et. al., Proc Natl Acad Sci USA. 81(11): 3332-3336, 1984).
As a vector that can be used in yeast, YEp352 and pRS315 are available from ATCC (The American Type Culture Collection, 10801 University Boulevard, Manassas, VA 20110, United States of America) as ATCC 37673 and ATCC 77144, respectively.

Introduction of an inducible promoter into yeast can be performed as follows. A plasmid containing a promoter region connected to the above-described marker gene is used as a template, and primers are designed at each end of a region containing the promoter and marker gene, and about 50 bp of sequences of the both ends of a promoter region of an enzyme in the ergosterol biosynthetic pathway that is to be inducibly expressed is added to the both ends of the primers. PCR is performed using these primers and a template plasmid, and the resultant PCR fragment is used to transform yeast by the lithium acetate method. Selection is performed based on the marker gene trait to select yeast in which the inducible promoter and marker gene have been integrated into a promoter region of the enzyme in the ergosterol biosynthetic pathway on a chromosome. Insertion of the inducible promoter into a predetermined position can be confirmed by PCR using a primer designed in the inserted fragment and a primer designed in a region of a gene of the enzyme in the ergosterol biosynthetic pathway or adjacent region on a chromosome in combination.

In a method of the present invention, first, a gene is introduced into yeast in the state where expression of an enzyme in the ergosterol biosynthetic pathway is induced.
For example, introduction of a gene into yeast in the state where expression of the enzyme in the ergosterol biosynthetic pathway is induced can be performed as follows. That is, in the case of yeast strain that expresses an enzyme in the ergosterol biosynthetic pathway by an inducible promoter, the yeast is cultured under a condition in which expression of an enzyme in the ergosterol biosynthetic pathway is induced, for example, in the case of GAL10 promoter, in a medium containing 2% galactose, to thereby induce expression of the enzyme in the ergosterol biosynthetic pathway. Alternatively, in the case of CUP 1 promoter, the yeast is cultured in a medium containing 0.5 mM CuSO₄, to thereby induce expression of the enzyme in the ergosterol biosynthetic pathway, or in the case of PHO5 promoter, the yeast is cultured in a low-phosphate medium containing 0.03 g/L KH₂PO₄, to thereby induce expression of the enzyme in the ergosterol biosynthetic pathway. Meanwhile, in the case of nmtl promoter, the yeast is cultured in a medium containing no thiamine, to thereby induce expression of the enzyme in the ergosterol biosynthetic pathway.

Introduction of a gene can be performed using a plasmid vector replicable in yeast cell. Introduction of a plasmid vector can be performed by the electroporation method or the lithium acetate method.
The type of a gene to be introduced is not particularly limited, and a gene library such as a cDNA library is preferably introduced.

Next, a compound is added to the yeast in the state where expression of the enzyme in the ergosterol biosynthetic pathway is repressed to allow the compound to interact with a protein expressed from the above-described introduced gene. The type of the compound is not particularly limited, and there may be used low-molecular compounds, peptides, naturally-occurring compounds, and the like, as well as drugs and toxicants.
The interaction of the compound in the state where expression of an enzyme in the ergosterol biosynthetic pathway is repressed may be performed as follows. That is, yeast is cultured under a condition which does not cause expression of an enzyme in the ergosterol biosynthetic pathway, for example, in the case of GAL10 promoter, in a medium containing no galactose, and the yeast is brought into contact with a compound under a condition in which expression of the enzyme in the ergosterol biosynthetic pathway is repressed to allow the compound to interact with a gene product of the introduced gene. In the case of CUP1 promoter, the yeast is cultured in a medium containing no CuSO₄, or in the case of PHO5 promoter, the yeast is cultured in a high-phosphate medium containing 0.3 g/L KH₂PO₄, and the yeast is brought into contact with the compound under a condition in which expression of the enzyme in the ergosterol biosynthetic pathway is repressed to allow the compound to interact with a gene product of the introduced gene. Meanwhile, in the case of nmt1 promoter, the yeast is cultured in a medium containing 5 mg/ml thiamine, and the yeast is brought into contact with the compound under a condition in which expression of the enzyme in the ergosterol biosynthetic pathway is repressed to allow the compound to interact with a gene product of the introduced gene.

Identification of a gene encoding a protein that reacts with a compound can be performed as follows: yeast whose phenotype has been altered by the interaction of the compound with a protein is selected from yeasts to which the compound is added, and a gene that has been introduced into the selected yeast is identified.

The phrase "allowing a compound to interact with a protein" includes allowing the compound to bind the protein, allowing the compound to interact with the protein to alter the activity of the protein, and allowing the binding to interact with the protein to cause a structural change of the compound. For example, it includes allowing a compound that serves as an antagonist or agonist of the protein to interact with a protein to alter the activity of the protein.

A phenotype of yeast is not limited as long as it is identifiable phenotype, and examples thereof include size, shape, color, etc. of a colony, and growth inhibition and lethality are preferable. The growth inhibition can be confirmed by the size of the colony, while the lethality can be confirmed by the presence or absence of growth.
For example, when a compound to be used is one that causes growth inhibition or lethality of yeast, yeast that can grow after allowing the compound to interact with a protein is selected.
Meanwhile, in the case of identifying a protein that binds to a compound, three-hybrid system as described below is used to select yeast that exhibits a phenotype indicated by a reporter gene based on the binding of the protein and the compound. More specifically, when the reporter gene is LacZ, yeast that exhibits blue color is selected, while, when the reporter gene is HIS3, yeast that grows in a histidine-deficient medium is selected.
In the case of identifying a protein that causes a structural change of a compound, yeast is selected based on a phenotype accompanied by the structural change of the compound.
Subsequently, the introduced gene is isolated from the yeast that has been selected. If the gene has been introduced as a plasmid, plasmids are isolated, and the nucleotide sequence is determined, to thereby identify a gene encoding a protein that has interacted with the compound.

One embodiment is application of the method of the present invention to the multicopy suppressor method. That is, the method is intended to select a gene encoding a protein that interacts with a compound in a gene library by: introducing a gene library into yeast in the state where an enzyme in the ergosterol biosynthetic pathway is expressed; allowing a compound to interact in the state where an enzyme in the ergosterol biosynthetic pathway is not expressed; selecting yeast whose phenotype caused by the compound is repressed; and identifying a gene that is introduced into the yeast.

This embodiment may be applied to the three-hybrid system (Edward J. Licitra and Jun O. Liu, Proc. Natl. Acad. Sci. USA, 93(23), 12817-12821, 1996) that is used for detecting an interaction between a compound and a protein based on the two-hybrid system that is used for detecting an interaction between two types of proteins. In the three-hybrid system, a fusion protein of a transcriptional activation domain of a transcription factor such as GAL4 with a protein X that is a target of a compound B to be detected, and a fusion protein of a DNA-binding domain of the transcription factor with a protein Y are expressed in a cell, while a DNA comprising a nucleotide sequence/promoter region to which the DNA-binding domain binds that is followed by a reporter gene is introduced. When a compound prepared by covalently linking a compound A that interacts with the protein Y with a compound B to be detected is allowed to interact, the protein Y and the compound A, and the protein X and the compound B are linked to each other, to thereby shift the transcriptional activation domain fused with the protein X to the neighborhood of the reporter gene, resulting in increase in expression of the reporter gene product. That is, the method is intended to select a gene encoding a protein X that is a target of the compound by: introducing a gene library of the three-hybrid system into yeast in the state where an enzyme in the ergosterol biosynthetic pathway is expressed; allowing a compound to interact in the state where an enzyme in the ergosterol biosynthetic pathway is not expressed; selecting yeast that expresses a reporter gene product; and identifying a gene that is introduced into the yeast.

Meanwhile, another embodiment of the present invention is application of the method of the present invention to the bioconversion system. That is, the method is intended to obtain a derivative of a compound by: introducing a gene into yeast in the state where an enzyme in the ergosterol biosynthetic pathway is expressed; allowing a compound to interact in the state where an enzyme in the ergosterol biosynthetic pathway is not expressed; and converting the compound by a gene product of the introduced gene.

Another embodiment is yeast that has an enzyme in the ergosterol biosynthetic pathway, preferably Erg3p and Erg6p in an inducible state.

### EXAMPLES

Hereinafter, the present invention will be described by referring to specific examples, but it is not limited thereto.

### Example 1: Creation of yeast that inducibly expresses ERG6

### (1) Construction of a template plasmid for GAL10 promoter:

A kanMx6 gene as a phenotypic marker was connected to GAL10 promoter that had been amplified by PCR, to thereby construct a GAL10 promoter template plasmid.
GAL10 promoter was amplified by PCR using yeast genomic DNA as a template, primers (SEQ ID NOS: 1 and 2) designed by adding a BamHI cleavage site to a region between GAL10 gene and GAL1 gene adjacent thereto, and Pfu DNA Polymerase (Promega).
NotI-digested fragment (containing kanMX6 gene) of pFA6a-kanMX6 (Wach A, et al., Yeast, 13(11): 1065-1075, 1997) was ligated to a NotI cleavage site in a pRS315 vector, and then, the obtained vector was cleaved with BamHI and Bg1II. Then, the above-described PCR-amplified GAL10 promoter fragment was treated with BamHI and inserted into the vector.
SEQ ID NO: 1: CGGGATCCCG TATAGTTTTT TCTCCTTGAC
SEQ ID NO: 2: CGGGATCCCG TTATATTGAATTTTCAAAAATT

### (2) Insertion of GAL10 promoter:

GAL10 promoter was inserted into an ERG6 promoter region in yeast *(Saccharomyces cerevisiae)* in which PDR1 and PDR3 are disrupted in accordance with a method of efficiently creating a gene-disrupted allele by PCR (Baudin A. et. al. Nucl. Acid Res. 21: 3329-3330, 1993).
Primers (SEQ ID NOS: 3 and 4) comprising about 50 bp of the sequence of the respective end of the ERG6 promoter region that is added to the sequence of the end of the GAL10 promoter or kanMx6 gene of the above-described template plasmid were designed. These primers and the template plasmid were used to perform PCR using TaKaRa Ex Taq, and the resultant PCR fragment was used to transform yeast by the lithium acetate method, followed by selection in a medium containing Geneticin (manufactured by Sigma), to thereby select yeast in which GAL10 promoter and KanMx6 gene had been integrated into an ERG6 promoter region on a chromosome.
Insertion of GAL10 promoter into a predetermined position was confirmed by PCR using a primer designed in the inserted fragment and a primer designed in the region of ERG6 gene or adjacent region on a chromosome, in combination.

SEQ ID NO: 3:
CTGTCTTTTTCTCAATTCTGTTTCACTCATCTTATGCTGCCTACTATATT-GATATCGAA
TTCCTGCAGCC

SEQ ID NO: 4:
CCTCCAATACTTGCTGTTGCCGATAACTTCTTCATTGCTTTTATATAGTGCATAGGC
CACTAGTGGATCTG

### Example 2: Drug resistance of yeast that inducibly expresses ERG6

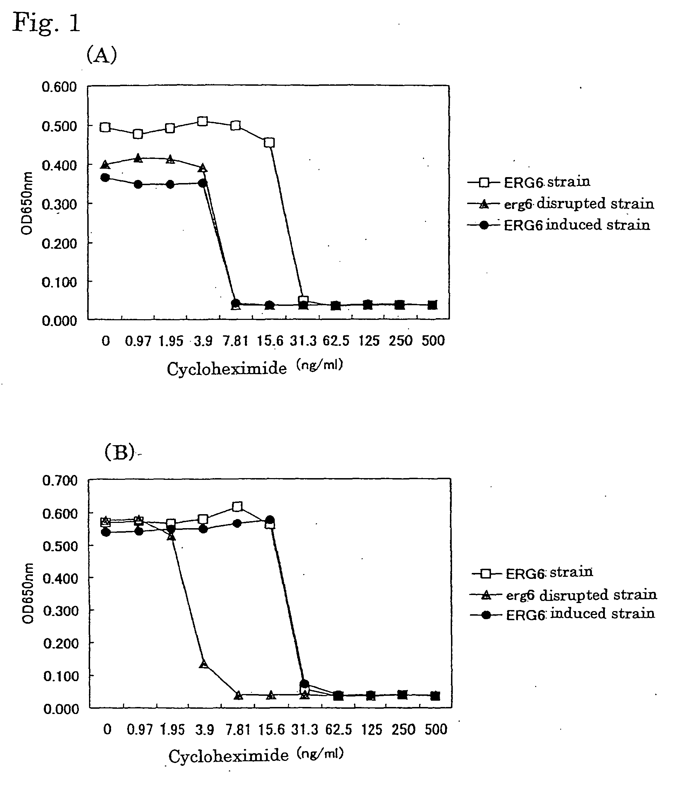

The following experiments were performed to confirm a difference in drug resistance of yeast that inducibly expresses ergosterol depending on presence or absence of induction.
The strain that inducibly expresses ERG6 that had been prepared in Example 1 was cultured at 30°C for 2 days in a glucose medium as well as in a galactose medium both of which contained cycloheximide in various concentrations, and the cycloheximide sensitivity of the strain that inducibly expresses ERG6 was determined. As a result, the sensitivity in the galactose medium was found to be equal to that of a pdr1 pdr3-disrupted stain (ERG6 strain), while the sensitivity in the glucose medium was found to be equal to that of a pdr1 pdr3 erg6-disrupted strain (Fig. 1). It was confirmed that the drug sensitivity was increased by turning off the expression of ergosterol.

### Example 3: Transformation of yeast that inducibly expresses ERG6

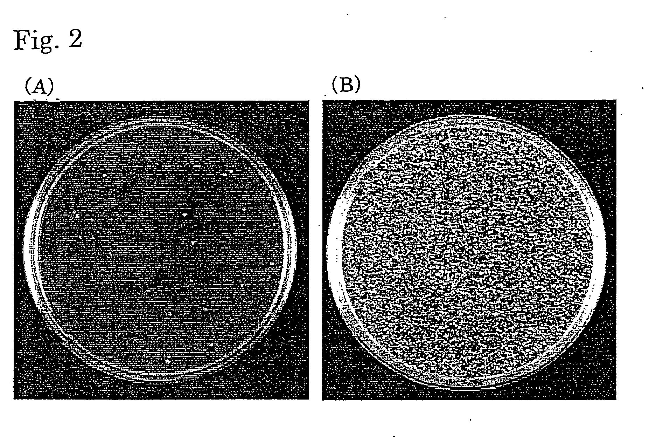

The following experiments were performed to confirm a difference in the transformation efficiency of yeast that inducibly expresses ergosterol depending on presence or absence of induction. The transformation was performed using YEp352GAPII, which was created by inserting GAPDH promoter and GAPDH terminator derived from pKT10 (Tanaka et al, Mol. Cell Biol., 10:4303-4313, 1990) into a multi-cloning site of YEp352 and then replacing the multi-cloning site with a pUC18 multi-cloning site.
The strain that inducibly expresses ERG6 that had been prepared in Example 1 was cultured in a liquid medium containing glucose or a liquid medium containing galactose, followed by transformation with the YEp352GAPII vector by the lithium acetate method.
The YEp352GAPII vector has URA3 as a selection marker, so that yeast into which the YEp352GAPII vector has been introduced forms a colony on SD(-ura) agar medium. The strain was cultured at 30°C for 2 days, and the colonies were counted. As a result, the number of colonies obtained in the medium containing galactose was found to be 700 times or more greater than that obtained on the medium containing glucose (Fig. 2). It was confirmed that the transformation efficiency was increased by expressing ergosterol.

### Example 4: Multicopy suppressor screening of methotrexate (MTX)

Methotrexate (MTX) is an analogue of dihydrofolic acid, and is known to interact with dihydrofolate reductase (DHFR) to inhibit synthesis of folic acid and has a growth inhibitory action against yeast. It was examined whether the multicopy suppressor method using yeast that inducibly expresses ERG6 can identify a dihydrofolate reductase gene as a target gene of MTX. A library prepared from HeLa cDNA was used as a human cDNA library.
The human cDNA library was used to transform the strain that inducibly expresses ERG6 that had been prepared in Example 1 by the lithium acetate method, and the resultant transformants (about 4.5 x 10⁶ cells) were collected. Then, the cells (5 x 10⁷ cells) were inoculated on an agar medium containing 25 µM MTX and cultured at 30°C for 3 days, to thereby form MTX-resistant colonies. Plasmids were collected from the resistant colonies, and sequencing was performed to determine sequences in the plasmids and revealed that the plasmids contained a target gene of MTX, i.e., the ORF of DHFR.
It has been reported that DHFR (DFR1 in budding yeast) imparts MTX-resistance (Lagosky PA. et. al. Nucleic Acids Res. 15(24): 10355-10371, 1987), and it was proved that DHFR is isolated by the multicopy suppressor screening using the strain that inducibly expresses ERG6, like the report.

### INDUSTRIAL APPLICABILITY

Yeast that has high drug permeability and maintains high transformation efficiency was obtained, thereby, the yeast has made it easy to search a drug target gene.

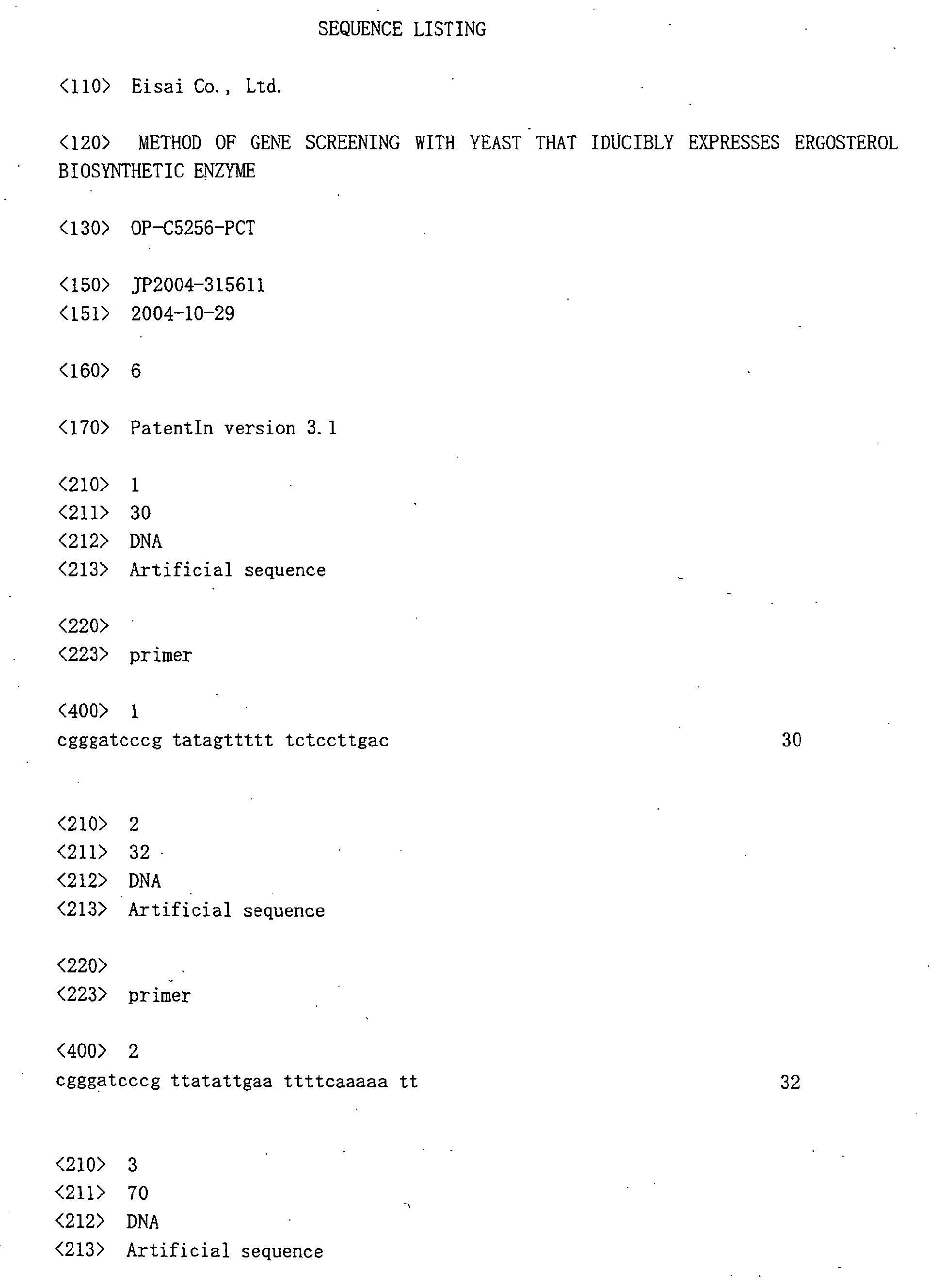

## Claims

1. A method of allowing a compound to interact with a protein in yeast cell, comprising the steps of:
(1) introducing a gene encoding the protein into yeast to express the protein in a state where expression of an enzyme involved in the ergosterol biosynthetic pathway is induced; and
(2) adding the compound to the yeast into which the gene has been introduced to allow the compound to interact with the protein in yeast cell in a state where expression of the enzyme in the ergosterol biosynthetic pathway is repressed.

2. A method of identifying a gene encoding a protein that interacts with a compound by contacting the compound with the protein expressed in yeast, comprising the steps of:
(1) introducing a gene library into yeast to express proteins in a state where expression of an enzyme in the ergosterol biosynthetic pathway is induced;
(2) adding the compound to the yeast where the gene library has been introduced to contact the compound with the protein in a state where expression of the enzyme in the ergosterol biosynthetic pathway is repressed;
(3) selecting yeast whose phenotype has been altered by the interaction between the compound and the protein; and
(4) identifying a gene that has been introduced into the selected yeast.

3. The method according to Claim 2, wherein the phenotype of yeast caused by the compound is lethality or growth inhibition.

4. The method according to any one of Claims 1 to 3, wherein the induction and the repression of expression of the enzyme in the ergosterol biosynthetic pathway is controlled by transcriptional regulation in the yeast.

5. The method according to Claim 4, wherein the transcriptional regulation is transcriptional regulation by GAL10 promoter or GAL1 promoter.

6. The method according to any one of Claims 1 to 5, wherein the enzyme in the ergosterol biosynthetic pathway is selected from ERG6 and ERG3.

7. Yeast which inducibly expresses ERG6 or ERG3.
